(19)

Europäisches Patentamt
European Patent Office
Office européen des brevets

(11) **EP 1 842 586 A1**

(12) **EUROPEAN PATENT APPLICATION**
published in accordance with Art. 158(3) EPC

(43) Date of publication:
**10.10.2007 Bulletin 2007/41**

(21) Application number: **05809275.0**

(22) Date of filing: **21.11.2005**

(51) Int Cl.:
*B01J 19/00* (2006.01)   *A61K 9/50* (2006.01)
*B01J 3/00* (2006.01)   *B01J 13/04* (2006.01)
*C08J 3/12* (2006.01)

(86) International application number:
**PCT/JP2005/021753**

(87) International publication number:
**WO 2006/057374 (01.06.2006 Gazette 2006/22)**

(84) Designated Contracting States:
**DE FR GB**

(30) Priority: **29.11.2004 JP 2004344934**

(71) Applicant: **Japan Science and Technology Agency**
**Kawaguchi-shi,**
**Saitama 332-0012 (JP)**

(72) Inventors:
• **MISHIMA, Kenji**
  **8110121 (JP)**
• **MATSUYAMA, Kiyoshi**
  **, Fukuoka 111345; (JP)**

(74) Representative: **Arth, Hans-Lothar**
  **Arth, Bucher & Kollegen,**
  **Am Klopferspitz 19 (IZB)**
  **82152 Martinsried (DE)**

(54) **METHOD FOR PREPARING COMPOSITE FINE PARTICLES**

(57)   The present invention provides a method for preparing composite microspheres of a high-molecular material and a core substance. This method includes the steps of: dissolving a high-molecular material and dispersing a core substance in a high pressure fluid containing a supercritical fluid and an entrainer, under a shear stress of 1 Pa or more; and spraying the resultant high pressure fluid containing the high-molecular material and the core substance into a poor solvent to cause rapid expansion. According to the method of the present invention, composite microspheres having a uniform size of several micrometers or less, and more preferably nanometer order (a size of 1 $\mu$m or less) can be obtained.

Fig. 1

EP 1 842 586 A1

**Description**

Technical Field

The present invention relates to a method for preparing composite microspheres of a high-molecular material and a core substance. More specifically, the present invention relates to a method for preparing composite microspheres having a uniform size of several microns to nanometer order.

Background Art

When preparing high-molecular weight microspheres or microcapsules containing a core substance such as inorganic particles, dispersion polymerization or emulsion polymerization using surfactants, emulsions or the like is performed (see Japanese Laid-Open Patent Publication No. 56-76447). However, in order to isolate microspheres obtained by such techniques, it is necessary to wash the microspheres to remove surfactants, emulsions, and moisture remaining on the surface of the microspheres, and then to dry the microspheres. Thus, there is a problem in that in this process, the microspheres adhere to each other to form large particles or blocks.

In order to solve this problem, a method for preparing high-molecular weight microspheres using a high pressure fluid such as supercritical carbon dioxide has been developed (see Japanese Laid-Open Patent Publication No. 8-104830). It is known that supercritical carbon dioxide has a poor solubility to a high-molecular material, which is a disadvantage of the supercritical carbon dioxide. This method has been developed based on thermodynamical examination that the low dissolving ability of supercritical carbon dioxide can be improved by addition of a poor solvent by a factor of 1000 or more. The effect by the addition of a poor solvent is called as a specific coexistence effect of a poor solvent. More specifically, it is possible to prepare high-molecular weight microspheres by using ethanol or the like, which is inherently a poor solvent with respect to a certain type of high-molecular materials, as an entrainer to increase the solubility of a high-molecular material specifically only when a supercritical fluid is mixed with the entrainer, and then by rapidly expanding this mixture under reduced pressure. In this method, the supercritical fluid is separated from the entrainer in the course of rapid expansion of the high pressure fluid containing dissolved high-molecular materials under reduced pressure, so that the solubility of the high-molecular materials is decreased rapidly and microspheres are produced. Furthermore, since the entrainer itself is volatile and is a poor solvent with respect to high-molecular materials, the extent of adherence between the high-molecular weight microspheres produced in the rapid expansion process is small.

Furthermore, a method for coating a core substance such as a protein, a pharmaceutical agent, organic microspheres, inorganic microspheres, a liquid substance and their mixture, with a high-molecular organic compound such as acrylic resin, an organic-inorganic compound or a mixture of these compounds and other compounds, by utilizing the above-described specific coexistence effect of a poor solvent has been also developed (see Japanese Laid-Open Patent Publication No. 11-197494). In this method, substances as described above are dissolved in a high pressure fluid containing a supercritical fluid and an entrainer, and this mixture is rapidly expanded into the air, so that coated microspheres are produced.

However, in the method described in Japanese Laid-Open Patent Publication No. 11-197494, it is possible to cover (coat) particles with a size of several micrometer order with high-molecular materials, but it is not possible to coat inorganic particles having a size of nanometer order. Usually, a homogenizer, a ball mill, or the like is used in order to disperse inorganic particles having a size of several micrometers or less in a liquid solvent. However, in a high pressure fluid such as a supercritical fluid, it is difficult to introduce a general purpose homogenizer or ball mill into a pressure vessel. Furthermore, it is also difficult to disperse particles by applying a shear stress to the particles using a special mechanical method as described in Japanese Laid-Open Patent Publication No. 2000-354751.

Disclosure of Invention

It is an object of the present invention to provide a method for preparing composite microspheres of a high-molecular material and a core substance, having a size of several micrometers or less, and more preferably having a size of nanometer order (a size of 1 $\mu$m or less).

The present invention provides a method for preparing composite microspheres of a high-molecular material and a core substance, the method comprises the steps of:

dissolving a high-molecular material and dispersing a core substance in a high pressure fluid containing a supercritical fluid and an entrainer, under a shear stress of 1 Pa or more; and
spraying the resultant high pressure fluid containing the high-molecular material and the core substance into a poor solvent to cause rapid expansion.

In one embodiment, the dissolving and dispersing step is performed using a high-speed agitating apparatus provided with a mechanical seal.

In another embodiment, the core substance is dispersed utilizing a shear stress of 1 Pa or more.

In a separate embodiment, in the composite microspheres, the core substance is coated with the high-molecular material.

In an embodiment, the core substance is inorganic particles.

In a further embodiment, the supercritical fluid is selected from the group consisting of carbon dioxide, ammonia, methane, ethane, ethylene, butane, and propane, the entrainer is at least one selected from the group consisting of water, methanol, ethanol, propanol, acetone, and a mixture thereof, and the poor solvent is at least one selected from the group consisting of water, methanol, ethanol, propanol, acetone, liquid nitrogen, and a mixture thereof.

According to the method of the present invention, the high-molecular material is dissolved and the core substance is dispersed in the high pressure fluid containing, for example, the supercritical carbon dioxide, using the high-speed agitating apparatus, and the resultant is rapidly expanded. Thus, it is possible to prepare composite microspheres of the high-molecular material and the core substance, having a size of several micrometers or less, and more preferably having a size of nanometer order. It is possible to prepare various composite microspheres depending on the combination of high-molecular materials and core substances by considering the solubility to the high pressure fluid. For example, when using a high-molecular material that is soluble to the high pressure fluid and a core substance (e.g., inorganic particles) that is insoluble to the high pressure fluid, it is possible to obtain composite microspheres in which the core substance is dispersed in the high-molecular material (more specifically, the core substance is coated with the high-molecular material).

Brief Description of Drawings

Fig. 1 is a schematic view of an apparatus used in the method for preparing composite microspheres of the present invention.

Fig. 2 is a scanning electron microphotograph of polylactic acid/titanium oxide composite microspheres prepared by the method of the present invention (Example 1).

Fig. 3 is a transmission electron microphotograph of polylactic acid/titanium oxide composite microspheres prepared by the method of the present invention (Example 1).

Fig. 4 is a particle size distribution diagram of polylactic acid/titanium oxide composite microspheres obtained by the method of the present invention (shear stress: 5 Pa) (Example 2).

Fig. 5 is a particle size distribution diagram of polylactic acid/titanium oxide composite microspheres obtained at low agitation speed (shear stress: 0.001 Pa) (Comparative Example 1).

Fig. 6 is a graph showing the relationship between the analysis results (intensity of rutile type titanium dioxide at 27°) obtained by using an X-ray diffraction apparatus and the shear stress caused by agitation, of polylactic acid/titanium oxide composite microspheres prepared by the method of the present invention.

Fig. 7 is a schematic view for illustrating shear stress.

Best Mode for Carrying Out the Invention

A method for preparing composite microspheres of the present invention includes the steps of dissolving a high-molecular material and dispersing a core substance in a high pressure fluid containing a supercritical fluid and an entrainer, under a shear stress of 1 Pa or more, and spraying the high pressure fluid into a poor solvent to cause rapid expansion. In the method of the present invention, for example, the core substance is dispersed as microspheres having a size of nanometer order in the high pressure fluid in which the high-molecular material is dissolved using a high-speed agitating apparatus, then the resultant is rapidly expanded, and thus composite microspheres made of the high-molecular material dissolved in the high pressure fluid and the core substance can be precipitated. Alternatively, by precipitating the high-molecular material around the dispersed core substance microspheres in a supersaturated state of the high-molecular material, composite microspheres in which the core substance is covered with the high-molecular material, that is, the core substance is coated with the high-molecular material, can be prepared.

(Composite Microspheres)

In this specification, "composite microspheres" refer to particles that are constituted by a high-molecular material and a core substance. The composite microspheres have a size of 10$\mu$m or less, and preferably a size of several micrometer order or less. Examples of the composite microspheres include composite microspheres in which a core substance is dispersed in a high-molecular material, that is, composite microspheres in which a core substance is covered (coated) with a high-molecular material.

(Supercritical Fluid)

In the present invention, a "supercritical fluid" refers to a fluid at a temperature higher than the critical temperature and under a pressure higher than the critical pressure, but may include a subcritical fluid. There is no particular limitation on the chemical species of such a supercritical fluid. For example, an organic gas in a gaseous state at ordinary temperature can be used. Carbon dioxide, carbon monoxide, ammonia, methane, ethane, propane, ethylene, and butane are preferable. Carbon dioxide, ammonia, methane, ethane, ethylene, and butane are more preferable, and carbon dioxide and ethylene are even more preferable.

(Entrainer)

In the present invention, an "entrainer" refers to a solvent that is added in order to improve the solubility of a high-molecular material for coating a core substance of targeted composite microspheres to a high pressure fluid. Usually, the entrainer is selected from poor solvents (described later in detail) with respect to a high-molecular material in use. The entrainer is selected preferably such that the solubility of the high-molecular material is low or extremely low in a fluid that is not a high pressure fluid (e.g., a fluid under the pressure in the course of rapid expansion, a fluid under ordinary pressure). Such an entrainer may be a solvent that is in a gaseous state or liquid state at ordinary temperature. For example, low-molecular weight chemical substances such as carbon dioxide, methane, ethane, ethylene, propane, butane, acetic acid, water, methanol, ethanol, and ammonia are preferable. In the present invention, water, methanol, ethanol, propanol, acetone, and their mixtures are more preferable.

(Poor Solvent)

In the present invention, a "poor solvent" refers to a solvent that has an ability of dissolving a high-molecular material but has a very low solubility thereto. Therefore, the poor solvent is selected as appropriate depending on the type of a high-molecular material used. As such a poor solvent, polar solvents are preferable and solvents that are in a liquid state at ordinary temperature are more preferable. Examples thereof include water; lower alcohols such as methanol, ethanol, and propanol; acetone; and low-molecular weight solvents such as acetic acid. Alternatively, liquid nitrogen can be also used. The entrainer and the poor solvent used in the method of the present invention may be the same or different.

(High Pressure Fluid)

In the present invention, a "high pressure fluid" refers to a fluid under supercritical to critical pressure, and particularly refers to a fluid containing a supercritical fluid and an entrainer in the present invention. The high pressure fluid is usually in a gaseous state, but may include a substance in a liquid state.

(High-molecular material)

In the present invention, a "high-molecular material" refers to a material that is used as a raw material for coating targeted composite microspheres. There is no particular limitation on the high-molecular material, and examples thereof include: polyamide (e.g., Nylon 6, Nylon 6-6), polyethylene, polypropylene, polybutene, polyethylene glycol, polypropylene glycol, polyvinyl alcohol, polyacrylamide, acrylic resins (polyacrylic acid; and polyacrylic ester such as polymethyl methacrylate), phenol resins, epoxy resins, silicone resins, polyurethane, polyester, polybutadiene, polystyrene, polytetrafluoroethylene, polylactic acid, polycarbonate, polyacetal, polysiloxane, dextran, gelatin, starch, celluloses (e.g., cellulose butyrate, cellulose nitrate), saccharides, chitins, polypeptide and a high-molecular copolymer having these substances as constituents, and a mixture containing these substances.

(Core Substance)

A "core substance" refers to a substance that can constitute composite microspheres when covered with a high-molecular material. The core substance may be an organic or inorganic substance that is insoluble to a high pressure fluid. The core substance is selected depending on the application of composite microspheres, and examples thereof include substances used for pharmaceuticals, food additives, and copying/recording/display, and substances used as materials of electronic elements and fuel cells.

Examples of organic substances suitable as the core substance include proteins (e.g., tuberactinomycin, polymyxin, insulin, lysozyme, α-chymotrypsin, pepsin, ovalbumin, serum albumin, amylase, lipase, casein); and dyes and coating materials (e.g., leuco dyes, shellac, maleic resins, acrylic resins, carbon blacks).

As inorganic substances suitable as the core substances, any inorganic substances can be used, such as sulfides, silicon compounds, metals, metal compounds, alkali metal compounds, and alkaline earth compounds that are known to those skilled in the electronic equipment field. Examples of such inorganic substances include: sulfides (e.g., zinc sulfide, cadmium sulfide, sodium sulfide); silicon compounds (e.g., silicon dioxide); metals (e.g., iron, nickel, cobalt, stainless steel, copper, zinc); oxides (e.g., iron oxide, titanium oxide, tungsten oxide, nickel oxide, cobalt oxide, molybdenum oxide, manganese oxide, copper oxide, tantalum oxide); metal compounds (e.g., ferrite, $MnFe_2O_4$, $MnFe_2O_4$, $ZnFe_2O_4$, $NiFe_2O_4$, $CuFe_2O_4$); and carbide (Pd-C, platinum-supported carbon Pt-C).

(High-speed Agitating Apparatus)

In the method of the present invention, shear stress is applied to the high-molecular material and the core substance in the high pressure fluid, and thus it is preferable to use an agitating apparatus that can provides agitation at high speed in order to obtain large shear stress. There is no particular limitation on the high-speed agitating apparatus, as long as it can be installed inside a pressure vessel and can stably disperse the core substance at a size of several micrometers or less, preferably a size of nanometer order. Examples of the agitating apparatus include an agitating apparatus provided with a mechanical seal. In the case of conventionally used magnetic induction agitating apparatuses, when agitation is performed at a high speed of several thousands rpm or more, a magnet placed inside the agitating apparatus generates heat due to an electromagnetic induction heating phenomenon. Thus, it is often difficult to perform stable agitation. Thus, in the present invention, an agitating apparatus provided with a mechanical seal is used, so that it is possible to stably perform agitation at a high speed of 5,000 rpm or more, preferably 10,000 rpm or more, and more preferably 15,000 rpm or more. With this high speed agitation, a shear stress of 1 Pa or more can be applied to the high pressure fluid that is present between an agitating blade and a wall face of an agitating vessel. Thus, particles of the core substance having a size of several micrometers or less, more preferably a size of nanometer order can be dispersed in the high pressure fluid.

Furthermore, in the high-speed agitating apparatus used in the present invention, the core substance is dispersed preferably utilizing a shear stress of 1 Pa or more that is generated between the agitating blade and the wall face of the agitating vessel. In order to effectively generate shear stress, the distance between the agitating blade and the wall face of the agitating vessel is 0.5 to 10 mm, and more preferably 1 to 5 mm.

By using such a high-speed agitating apparatus, aggregation of the core substance of nano-microspheres that are easily aggregated in the high pressure fluid, is prevented. Thus, the dispersed microspheres are supplied to the following rapid expansion step. Accordingly, dispersed composite microspheres can be prepared.

(Shear Stress)

In the present invention, using the above-described high-speed agitating apparatus, agitation is performed under a shear stress of 1 Pa or more. The shear stress that is generated in the agitating apparatus is calculated as below.

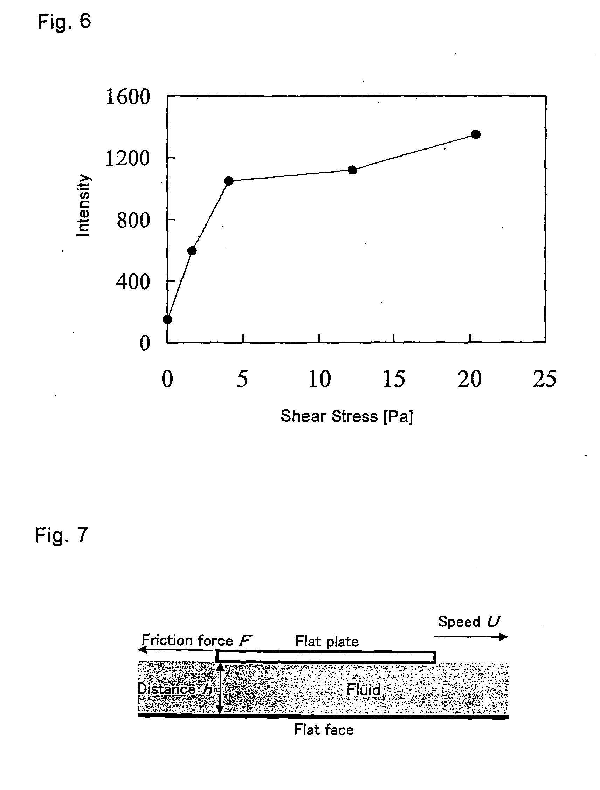

When a fluid is placed on a flat face, and a flat plate that is further placed thereon is slid (see Fig. 7), the frictional force F is proportional to the viscosity η of the fluid, the speed U of the flat plate, and the area A of the flat plate, and is inversely proportional to the distance h between the flat face and the flat plate, as indicated by Equation (1) below.

$$F = \frac{\eta\, UA}{h} \qquad (1)$$

Furthermore, the frictional force can be expressed as the product of the shear stress τ (frictional force per unit area) and the area A of the flat plate, as indicated by Equation (2) below.

$$F = \tau A \qquad (2)$$

From Equations (1) and (2) above, the shear stress τ is expressed by Equation (3) below.

$$\tau = \frac{\eta \, U}{h} \qquad (3)$$

Accordingly, the shear stress $\tau$ that is generated by agitation can be calculated by Equation (3).

It should be noted that the speed U (m/sec) is determined by the rotational speed of the apparatus and the diameter of an agitating cylinder. For example, if rotation is performed at a rotational speed n of 5000 rpm (83.3 rotations/second), then the circumferential speed U can be calculated as below. If the diameter d of the agitating cylinder is 0.156 m (radius r = 0.078 m), then the circumference L of the agitating cylinder is calculated as:

$$L = 2\pi r = 2 \times 3.14 \times 0.078 = 0.489 \text{ m.}$$

Accordingly, the circumferential speed U is obtained as:

$$U = n \times L = 83.3 \times 0.489 = 40.8 \text{ m/sec.}$$

(Method for Preparing Composite Microspheres)

In the method for preparing composite microspheres of the present invention, first, a high-molecular material is dissolved and a core substance is dispersed in a high pressure fluid containing a supercritical fluid and an entrainer, while agitating under a shear stress of 1 Pa or more. In this process, there is no particular limitation on the order of mixing the supercritical fluid, the entrainer, the high-molecular material, and the core substance. For example, a fluid containing a supercritical fluid and an entrainer is prepared in advance and then a high-molecular material is dissolved and a core substance is dispersed therein. Alternatively, an entrainer, a high-molecular material, and a core substance are mixed in advance, and then a supercritical fluid is added thereto, so that the high-molecular material is dissolved and the core substance is dispersed therein. In this case, the high-molecular material and the core substance may be respectively dissolved and dispersed in advance in a small amount of entrainer.

In the present invention, the core substance is added in a ratio of about 0.01 to 2 parts by weight, and preferably about 0.1 to 1 part by weight, with respect to 1 part by weight of the high-molecular material. The supercritical fluid is used in a ratio of about 20 to 100 parts by weight, and preferably about 30 to 80 parts by weight, with respect to 1 part by weight of the high-molecular material. Furthermore, the entrainer is used in a ratio of about 1 to 100 parts by weight, and preferably about 10 to 50 parts by weight, with respect to 1 part by weight of the high-molecular material.

The pressure in this process is preferably 7.2 to 30 MPa, and more preferably 15 to 25 MPa in order to efficiently perform the following process of rapid expansion of the high pressure fluid. The temperature is preferably 273 to 353 K, and more preferably 298 to 313 K.

Using the high-speed agitating apparatus provided with the mechanical seal, the high pressure fluid containing the high-molecular material and the core substance is agitated at the critical temperature and under the critical pressure. Such a high speed agitation is performed under a shear stress of 1 Pa or more, preferably 2 Pa or more, and more preferably 5 Pa or more.

Next, the high pressure fluid containing the dissolved high-molecular material and the dispersed core substance is sprayed into the poor solvent to cause rapid expansion. In this process, there is no particular limitation on the manner in which the high pressure fluid is sprayed into the poor solvent. For example, the high pressure fluid under high pressure can be sprayed into the poor solvent using a nozzle or the like; or a surfactant or the like can be previously dissolved in the poor solvent, and then the high pressure fluid can be blown to the poor solvent. In view of the solubility of the high-molecular material and core substance used, the dispersibility of the composite microspheres can be made better by appropriately changing factors such as the type, the pressure, and the temperature of the poor solvent. The temperature of the poor solvent is preferably 273 to 353 K, and more preferably 298 to 313 K. Thus, composite microspheres having a comparatively uniform average particle size can be obtained.

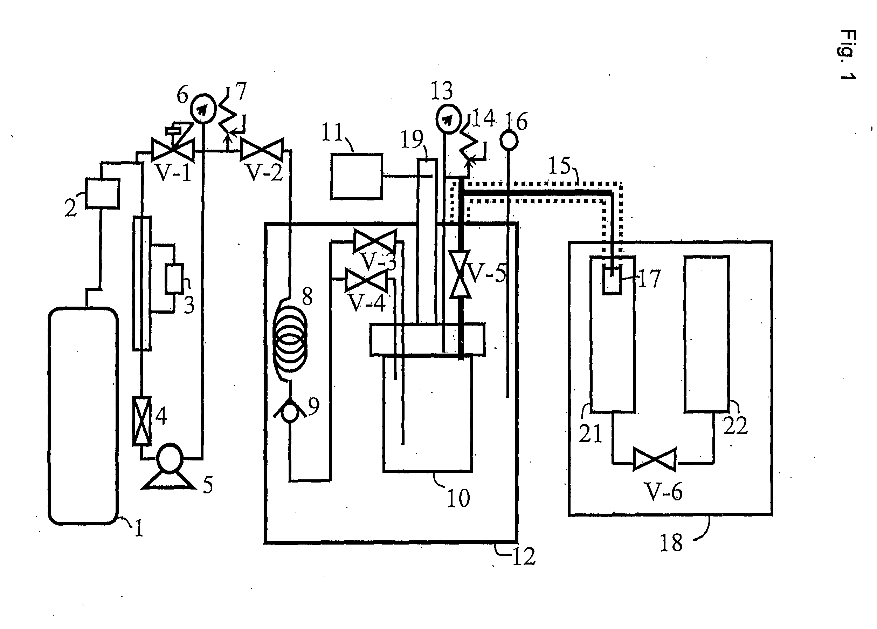

There is no particular limitation on the apparatus that can be used in the method of the present invention, as long as it is provided with means for agitating the high pressure fluid at high speed (e.g., a high-speed agitating apparatus provided with a mechanical seal) and means for spraying the high pressure fluid into the poor solvent, thereby causing rapid expansion (e.g., a nozzle). For example, the apparatus as shown in Fig. 1 can be used. Hereinafter, the method for preparing composite microspheres of the present invention is described more specifically with reference to Fig. 1.

The apparatus shown in Fig. 1 includes a pressure-rising portion that is from a cylinder 1 to a stop valve V-2, a mixing portion that is further downstream to a stop valve V-5, and a microsphere dispersing portion that contains a poor solvent for expanding rapidly and dispersing microspheres.

The pressure-rising portion is provided mainly with a cylinder (or tank) 1 for supplying the supercritical fluid (e.g., carbon dioxide) and a pressure-rising pump 5. If necessary, a tank for supplying the entrainer and another pressure-rising pump may be additionally provided.

A drying pipe 2, a cooling unit 3, and a filter 4 are provided between the cylinder 1 in which liquid carbon dioxide is filled and the pressure-rising pump 5. The liquid carbon dioxide from the cylinder 1 passes through the drying pipe 2, the cooling unit 3, and the filter 4, and is leaded to the pressure-rising pump 5 so as to increase its pressure. Then, the pressurized liquid carbon dioxide is sent to the mixing portion.

The drying pipe 2 is filled with a desiccant, and removes moisture from the liquid carbon dioxide that is passing therethrough. In the examples described below, a carrier gas drying pipe (Gas Driers: material; SUS316, the maximum use pressure; 20 MPa, inner diameter; 35.5 mm, length; 310 mm) manufactured by GL Science Inc. is used as the drying pipe 2, and a molecular sieve 5A (1/16 inch pellet) manufactured by GL Science Inc. is used as the desiccant.

The cooling unit 3 is filled with ethylene glycol, for example, and is constituted such that this ethylene glycol is cooled to about 260 K. The liquid carbon dioxide in which moisture has been removed by the drying pipe 2 is cooled by the ethylene glycol. In the examples described below, as the cooling unit 3, BL-22 manufactured by Yamato Scientific Co., Ltd. is used.

The filter 4 is provided after the cooling unit 3. The filter 4 removes contaminants such as dust so that the contaminants are prevented from entering into the pressure-rising pump 5. In the examples described below, as the filter 4, a filter having an average pore size of about 10 $\mu$m (FT4-10 manufactured by GL Science Inc.) is used.

In the examples described below, as the pressure-rising pump 5, a high pressure single plunger pump APS-5L (the maximum pressure: 58.8 MPa, normal pressure: 49.0 MPa, flow rate: 0.5 to 5.2 ml/min) manufactured by GL Science Inc. is used. A cooler is provided in the head portion of the pressure-rising pump 5 in order to prevent vaporization of the liquid carbon dioxide.

Furthermore, a pressure-regulating valve V-1 is provided in the pressure-rising portion, and this pressure-regulating valve V-1 sets the pressure in the system of the pressure-rising portion and the mixing portion to an arbitrary pressure. In the examples described below, as the pressure-regulating valve V-1, 26-1721-24 manufactured by TESCOM Corporation is used. The pressure-regulating valve V-1 can control the pressure in the system at a precision within $\pm$ 0.1 MPa, and the maximum use pressure is 41.5 MPa.

A pressure gauge 6 is provided in the pressure-rising portion, and this pressure gauge 6 is used for measuring the pressure in the system. The pressure gauge 6 is provided with an upper limit contact output terminal, and is set such that the power of the pressure-rising pump 5 is turned off at a specified pressure. In the examples described below, as the pressure gauge 6, a Bourdon pressure gauge LCG-350 (the maximum use pressure: 34.3 MPa) manufactured by GL Science Inc. was used. Economy Pressure Gauge PE-33-A (strain gauge, precision $\pm$ 0.3%) manufactured by Sokken Co., Ltd. was used to calibrate the pressure gauge 6.

Although not shown in the figures, in a case where an entrainer tank is provided, a pressure-rising pump, a check valve, and a stop valve are arranged between the entrainer tank and a high pressure cell 10. The entrainer (ethanol) filled in the entrainer tank passes through the check valve by the pressure-rising pump and is supplied to the high pressure cell 10, and then is mixed with the carbon dioxide supplied from the cylinder 1. In a case where an entrainer tank is not provided, the entrainer may be mixed with the supercritical fluid in advance, or may be supplied together with the high-molecular material to the high pressure cell 10 (described later in detail) of the mixing portion.

The stop valve V-2 is disposed between the pressure-rising portion and the mixing portion, and this stop valve V-2 can control the flow of the fluid to the mixing portion. In the examples described below, as the stop valve V-2, 2 Way Valve 02-0120 (the maximum use pressure: 98.0 MPa) manufactured by GL Science Inc. is used.

Furthermore, a safety valve 7 is provided between the pressure-rising portion and the mixing portion in order to ensure safety. In the examples described below, a spring safety valve manufactured by NUPRO was used as the safety valve 7, and a stainless steel pipe was used as the pipe.

The mixing portion is provided mainly with a thermostatic water chamber 12, as well as a preheating column 8, a check valve 9, and the high pressure cell 10 provided with a high-speed agitating apparatus 11, which are arranged in the thermostatic water chamber 12.

The thermostatic water chamber 12 is constituted so as to control the temperature of the high pressure cell 10 disposed therein. In the examples described below, the temperature is controlled using a temperature controller DB1000 of CHINO corporation. This temperature controller can control the water temperature within $\pm$ 0.1°C. A platinum resistance thermometer 1TPF483 manufactured by CHINO corporation was used as a temperature-measuring portion 16.

A mixture of the fluid (e.g., carbon dioxide) and the entrainer (e.g., ethanol) supplied from the pressure-rising portion is sent to the preheating column 8, where the mixture is heated from a temperature that is not higher than the critical temperature to the critical temperature or higher so that the fluid is turned into the supercritical fluid (fluid at the critical temperature or higher). This mixture is then introduced to the high pressure cell 10 by regulating stop valves V-3 and V-4. The check valve 9 is provided between the preheating column 8 and the stop valves V-3 and V-4. In the examples described below, as the check valve 9, SS-53F4 (the maximum use pressure: 34.3 MPa) manufactured by AKICO Corporation is used.

In the examples described below, as the high pressure cell 10, a quick openable extraction cell (material: SUS316, design pressure: 39.2 MPa (400 kg/cm$^2$), design temperature 423.15 K (150°C), inner diameter: 55 mm, height: 110 mm, inner volume: 250 ml) manufactured by AKICO Corporation is used.

Usually, the high-molecular material and the core substance are charged into the high pressure cell 10 in advance. If necessary, the entrainer also may be charged in advance. The supercritical fluid is added to this mixture, and the high pressure fluid is agitated at high speed using the high-speed agitating apparatus 11 sealed with a mechanical seal 19. The agitation speed is usually 5000 to 15000 rpm. The rotational speed of the agitating shaft is displayed by a digital rotation display meter, for example. In the examples described below, as the high-speed agitating apparatus 11 provided with the mechanical seal, 45/40-B023R4sp manufactured by TANKEN SEAL SEIKO CO., LTD was used. In the examples described below, the pressure inside the high pressure cell 10 is measured by a Bourdon pressure gauge E93004 6B (not shown) (the maximum pressure: 49.0 MPa) manufactured by Yamazaki Keiki Seisakusho. For calibration of this pressure gauge, Economy Pressure Gauge PE-33-A (strain gauge, precision $\pm$ 0.3%FS, FS: kgf/cm$^2$) manufactured by Sokken Co., Ltd. is used.

A safety valve 14 may be provided above portion of the high pressure cell 10 for the purpose of preventing the high pressure cell 10 from being exploded due to the pressure rise inside the high pressure cell 10. In the examples described below, as the safety valve 14, a spring safety valve (177-R3AKI-G) manufactured by NUPRO is used.

The microsphere dispersing portion followed by the mixing portion has a poor solvent cell 21 containing a poor solvent and a pressure buffering cell 22 in an air thermostatic chamber 18. The high pressure fluid in which the high-molecular material is dissolved and the core substance is dispersed passes via the valve V-5 through a protective pipe 15. Then, the high pressure fluid is sprayed from a nozzle 17 into the poor solvent cell 21 containing a poor solvent (e.g., water) provided in the air thermostatic chamber 18 so that microspheres are dispersed in the poor solvent. The extra pressure generated in the poor solvent cell 21 can be buffered by the pressure buffering cell 22 that is in communication therewith via a stop valve V-6. The poor solvent cell 21 and the pressure buffering cell 22 are provided with heaters to adjust the temperature in the rapid expansion of the high pressure fluid sprayed into the poor solvent. The protective pipe 15 and the nozzle 17 are also provided with heaters to prevent condensation of the sample due to pressure reduction and occurrence of dry ice due to the supercritical fluid (carbon dioxide). In the examples described below, as cells for the poor solvent cell 21 and the pressure buffering cell 22, cells (SUS316, design pressure: 34.3 MPa, design temperature: 373.15 K (100°C), inner diameter: 45 mm, height: 161.5 mm, and inner volume: 250 ml) manufactured by GL Science Inc., was used. As the protective pipe 15, a 1/8 inch stainless steel pipe (SUS316, outer diameter 3.175 mm, inner diameter 2.17 mm, and length about 1 m) was used. The inner volume of the air thermostatic chamber 18 is 125 dm$^3$, and the temperature in the thermostatic chamber is controlled within $\pm$0.05°C by the temperature regulator DB1000 manufactured by CHINO corporation. As the nozzle 17, a unijet nozzle (orifice diameter: 0.28 mm, the maximum use pressure: 280 kg/cm$^2$) manufactured by Spraying Systems Co., Japan was used.

When the high pressure fluid in which the high-molecular material is dissolved and the core substance is dispersed is rapidly expanded in the poor solvent cell 21, the dissolved high-molecular material is precipitated in the poor solvent cell 21, and thus composite microspheres made of the high-molecular material and the core substance can be obtained. After the pressure inside the poor solvent cell 21 is reduced by opening the valve V-6, the poor solvent cell 21 and the pressure buffering cell 22 are opened. Then, the poor solvent in which the composite microspheres are dispersed is taken out. By removing the poor solvent by drying under reduced pressure, only the composite microspheres can be collected.

Examples

The present invention will be described more specifically by way of examples below, but the present invention is not limited thereto. It should be noted that in the examples below, a manufacturing apparatus provided with components such as the above-described devices and apparatuses was used.

(Example 1)

Using the apparatus shown in Fig. 1, composite microspheres constituted by titanium oxide covered with polylactic acid were prepared in the following manner.

First, 5 g of polylactic acid (molecular weight: 10000), 1 g of titanium oxide (average primary particle size: 35 nm), and 200 ml of ethanol serving as the entrainer were placed in the high pressure cell 10. Then, after the poor solvent cell 21 was filled with water as the poor solvent, the high pressure cell 10 and the poor solvent cell 21 were placed at respective predetermined positions.

Then, carbon dioxide was supplied from the cylinder 1 while the valve V-2 was closed. The upper limit pressure of the carbon dioxide was regulated by the pressure-regulating valve V-1. The temperature in the thermostatic water chamber 12 was regulated to 313.15 ± 0.2 K by the temperature regulator. The temperature in the protective pipe 15 was regulated to 350.15 ± 0.5 K. Then, while the valve V-5 in the mixing portion was closed, the valve V-3 or V-4 was opened so that the carbon dioxide was supplied to the mixing portion. The valve V-4 was opened and kept opened until the pressure reached a predetermined pressure (25 MPa) in the high pressure cell 10. The inside of the high pressure cell 10 was agitated by the high-speed agitating apparatus 11. The rotational speed of the agitating shaft was adjusted to 10,000 rpm with a digital rotation display meter, thereby setting the shear stress to 5 Pa, and the entire system was pressurized and adjusted to 25 MPa. After the pressure became constant, agitation was continued for further 1 minute.

Then, the valve V-5 was opened. While reducing the pressure from 25 MPa to 20 MPa, the high pressure fluid containing the dissolved polylactic acid and the dispersed core substance was rapidly sprayed from the nozzle 17 into the poor solvent cell 21 containing the poor solvent, so that the resultant composite microspheres were dispersed therein. At the same time, the valve V-6 was opened to buffer the pressure by the pressure-buffering cell 22, and then the composite microspheres were collected together with water, which was the poor solvent.

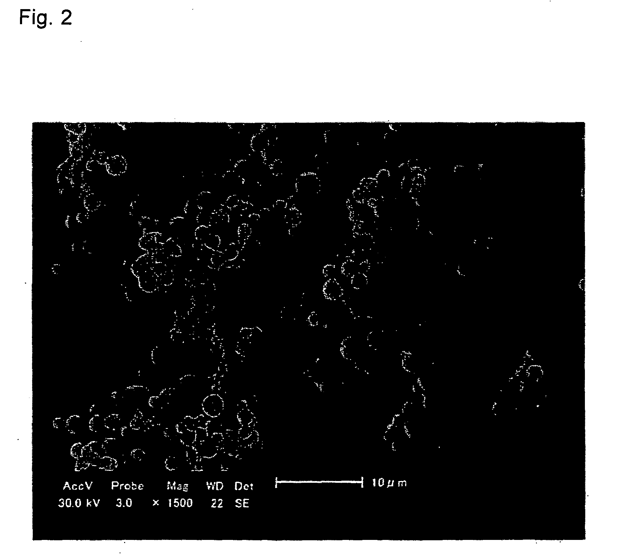

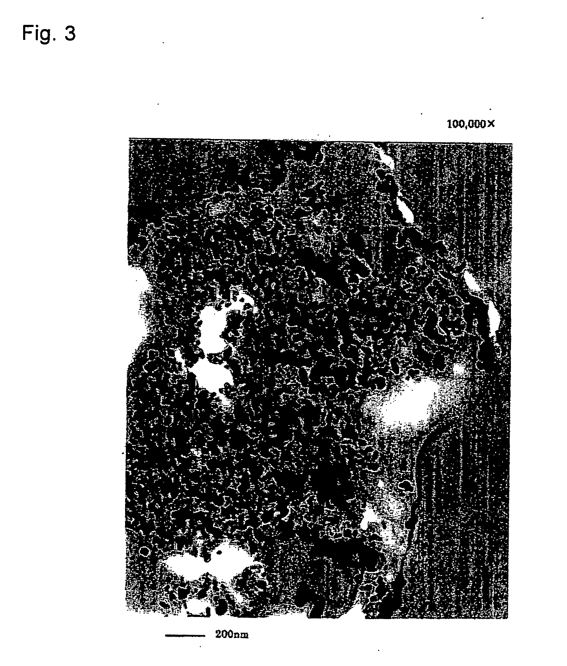

The obtained composite microspheres were observed through a scanning electron microscope (SEM-EDX) SSX-550 manufactured by Shimadzu Corporation and a transmission electron microscope (H-7100FA) manufactured by Hitachi, Ltd. The scanning electron microphotograph is shown in Fig. 2, and a transmission electron microphotograph is shown in Fig. 3. As shown in Fig. 2, a large number of polylactic acid microspheres having a diameter of several micrometers were obtained. Furthermore, as shown in Fig. 3, inside the obtained polylactic acid microspheres, titanium oxide particles were present in a dispersed state. That is to say, the titanium oxide was covered with the polylactic acid.

(Example 2)

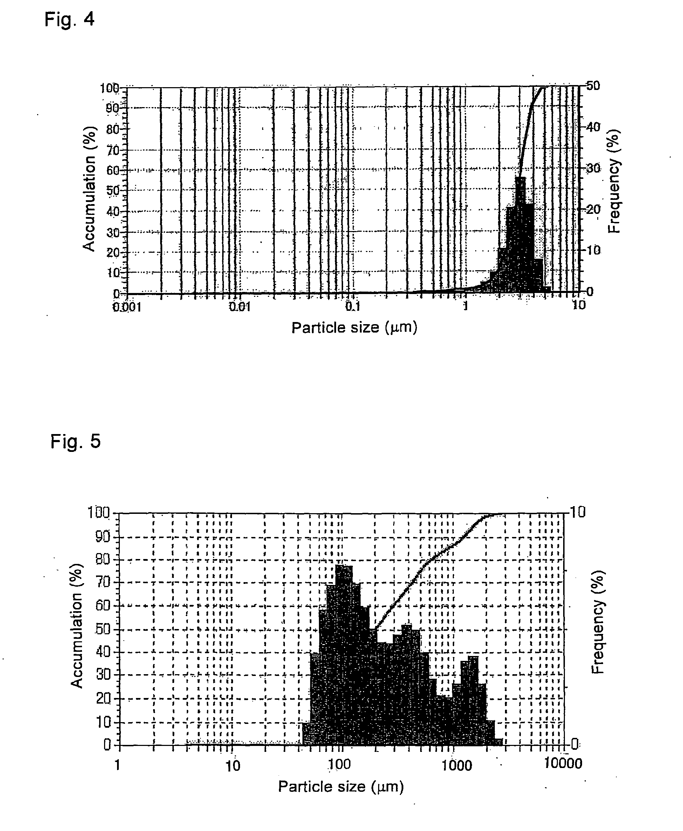

Polylactic acid/titanium oxide composite microspheres were prepared as in Example 1, except that titanium oxide having an average primary particle size of 100 nm was used, and that the pressure inside the high pressure cell 10 was 20 MPa. The particle size distribution of the obtained composite microspheres was measured with a particle size analyzer (Microtrac manufactured by NIKKISO CO., LTD.). The results are shown in Fig. 4. The obtained composite microspheres exhibited a normal distribution whose peak was at a particle size of about 4 $\mu$m. Thus, it was found that composite microspheres having a uniform average particle size were obtained.

(Comparative Example 1)

Polylactic acid/titanium oxide composite microspheres were prepared as in Example 1, except that titanium oxide having an average primary particle size of 100 nm was used, that the pressure inside the high pressure cell 10 was 20 MPa, and that the shear stress was set to 0.001 Pa by setting the rotation speed of the agitating apparatus to 1,000 rpm. The particle size distribution of the obtained composite microspheres was measured with the particle size analyzer (Microtrac manufactured by NIKKISO CO., LTD.) as in Example 2. The results are shown in Fig. 5. The composite microspheres obtained at low agitation speed (1,000 rpm) exhibited a polydispersed particle size distribution. It seems that this polydispersed distribution was obtained because titanium oxide particles were not sufficiently dispersed.

(Example 3)

Polylactic acid/titanium oxide composite microspheres were prepared as in Example 1, except that the shear stress was changed from 0 to 20 Pa. The amount of titanium oxide contained in the obtained composite microspheres was observed using an X-ray diffraction apparatus (198XHF-SRA manufactured by Mac Science). It was found that when the content of titanium dioxide was increased, the intensity of rutile type titanium dioxide at 27° was also increased. The results are shown in Fig. 6. As shown in Fig. 6, as shear stress increased in accordance with an increase in agitation speed, the intensity of rutile type titanium dioxide at 27° increased. Thus, it was found that the amount of titanium dioxide contained in the composite microspheres also increased.

Industrial Applicability

According to the method of the present invention, composite microspheres of a high-molecular material and a core substance, which have a uniform average particle size and a size of nanometer order, can be obtained. It is possible to prepare various composite microspheres in which the core substance is covered with the high-molecular material (that is, the surface of the core substance is coated), depending on the combination of the core substance and the high-molecular material. The nanometer-order composite microspheres of the high-molecular material and the core substance can be used in various applications, such as food, pharmaceuticals, cosmetics, microimage elements, toner, coating materials, catalyst carriers of fuel cells and the like, carriers for a filler of a separation column for liquid chromatography and the like, depending on the properties and functions of each of the high-molecular material and the core substance.

**Claims**

1.  A method for preparing composite microspheres of a high-molecular material and a core substance, comprising the steps of:

    dissolving a high-molecular material and dispersing a core substance in a high pressure fluid containing a supercritical fluid and an entrainer, under a shear stress of 1 Pa or more; and
    spraying the resultant high pressure fluid containing the high-molecular material and the core substance into a poor solvent to cause rapid expansion.

2.  The method of claim 1, wherein the dissolving and dispersing step is performed using a high-speed agitating apparatus provided with a mechanical seal.

3.  The method of claim 1 or 2, wherein the core substance is dispersed utilizing a shear stress of 1 Pa or more.

4.  The method of any one of claims 1 to 3, wherein in the composite microspheres, the core substance is coated with the high-molecular material.

5.  The method of any one of claims 1 to 4, wherein the core substance is inorganic particles.

6.  The method of any one of claims 1 to 5, wherein the supercritical fluid is selected from the group consisting of carbon dioxide, ammonia, methane, ethane, ethylene, butane, and propane.

7.  The method of any one of claims 1 to 6, wherein the entrainer is at least one selected from the group consisting of water, methanol, ethanol, propanol, acetone, and a mixture thereof.

8.  The method of any one of claims 1 to 7, wherein the poor solvent is at least one selected from the group consisting of water, methanol, ethanol, propanol, acetone, liquid nitrogen, and a mixture thereof.

Fig. 1

Fig. 2

AccV    Probe    Mag    WD   Det                        10 μm
30.0 kV   3.0    × 1500   22   SE

Fig. 3

100,000×

200nm

Fig. 4

Fig. 5

Fig. 6

Fig. 7

<div align="center">

**INTERNATIONAL SEARCH REPORT**

</div>

| | International application No. |
|---|---|
| | PCT/JP2005/021753 |

A. CLASSIFICATION OF SUBJECT MATTER
*B01J19/00*(2006.01), *A61K9/50*(2006.01), *B01J3/00*(2006.01),
*B01J13/04*(2006.01), *C08J3/12*(2006.01)

According to International Patent Classification (IPC) or to both national classification and IPC

B. FIELDS SEARCHED

Minimum documentation searched (classification system followed by classification symbols)
*B01J19/00*(2006.01), *A61K9/50*(2006.01), *B01J3/00*(2006.01),
*B01J13/04*(2006.01), *C08J3/12*(2006.01)

Documentation searched other than minimum documentation to the extent that such documents are included in the fields searched
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | JP 11-197494 A (Kenji MISHIMA),<br>27 July, 1999 (27.07.99),<br>Fig. 1; Claims; Par. Nos. [0026] to [0053]<br>(Family: none) | 1-8 |
| Y | JP 2004-130296 A (Kao Corp.),<br>30 April, 2004 (30.04.04),<br>Fig. 1; Par. Nos. [0073] to [0076]<br>(Family: none) | 1-8 |
| Y | JP 2004-82089 A (Kao Corp.),<br>18 March, 2004 (18.03.04),<br>Fig. 1; Par. Nos. [0073] to [0075]<br>& US 2002/0041927 A1 | 1-8 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 February, 2006 (14.02.06) | 28 February, 2006 (28.02.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

| International application No. |
| --- |
| PCT/JP2005/021753 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2004-503603 A  (SEPAREX),<br>05 February, 2004 (05.02.04),<br>Fig. 1; Par. No. [0038]<br>& US 2003/0157183 A1 | 1-8 |

Form PCT/ISA/210 (continuation of second sheet) (April 2005)

**EP 1 842 586 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 56076447 A
- JP 8104830 A
- JP 11197494 A
- JP 2000354751 A